(19) Europäisches Patentamt
European Patent Office
Office européen des brevets

(11) Publication number: **0 169 708 B1**

(12) **EUROPEAN PATENT SPECIFICATION**

(45) Date of publication of patent specification: **08.11.89**

(51) Int. Cl.[4]: **C 08 G 18/18**, C 08 G 59/68

(21) Application number: **85305120.9**

(22) Date of filing: **18.07.85**

(54) **Process for the formation of isocyanurates.**

(30) Priority: **27.07.84 US 635280**

(43) Date of publication of application: **29.01.86 Bulletin 86/05**

(45) Publication of the grant of the patent: **08.11.89 Bulletin 89/45**

(84) Designated Contracting States: **BE DE FR GB IT NL**

(56) References cited:
**DE-A-2 740 093**
**FR-A-2 215 447**
**FR-A-2 291 995**

**CHEMICAL ABSTRACTS, vol. 83, no. 22, 1st December 1975, page 127, abstract no. 181215f, Columbus, Ohio, US; & JP-A-75 59 426 (HITACHI LTD.) 22-05-1975**

(73) Proprietor: **BP Chemicals Limited**
**Belgrave House 76 Buckingham Palace Road**
**London, SW1W 0SU (GB)**

(72) Inventor: **Ashida, Kaneyoshi**
**4001 W. McNichols Road**
**Detroit Michigan 48221 (US)**

(74) Representative: **Fawcett, Richard Fennelly et al**
**BP INTERNATIONAL LIMITED Patents Division**
**Chertsey Road**
**Sunbury-on-Thames Middlesex, TW16 7LN (GB)**

**EP 0 169 708 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for preparing isocyanurates by the trimerisation of isocyanates and to a method for introducing isocyanurate linkages into condensation polymers in which one of the reactive monomers is an isocyanate. In particular, the invention relates to the use of a novel class of silicate catalysts which catalyse the formation of isocyanurates or the formation of isocyanurate linkages in isocyanate based condensation polymers.

US—A—4,072,626, GB—A—1,497,956 and US—A—4,133,781 disclose the addition of organic silicates to modified isocyanurate foams for the purpose of smoke suppression. However, no catalytic activity for these materials has been reported.

It has now been found that the silicates of the present invention not only act as a smoke suppressing agent but also catalyse the formation of isocyanurates from isocyanates.

Accordingly, the present invention provides a process for the preparation of an isocyanurate from an isocyanate or mixture thereof by addition to the isocyanate or mixture of isocyanates a silicate catalyst having the formula

$$Si(O-R_1-N(R_2)_2)_4 \qquad 1)$$

or

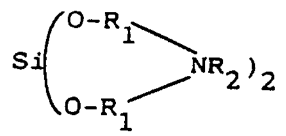

2)

wherein $R_1$ is a $C_2$—$C_4$ alkylene radical and

$R_2$ is an alkyl radical.

An embodiment of the present invention provides a process for the preparation of isocyanate condensation polymers containing isocyanurate linkages by addition to the mixture which produces the polymer, of a silicate catalyst of the type described herein.

The silicate catalysts may be of the general formula $Si(O—R_1—N(R_2)_2)_4$ or $Si((O—R_1)_2—NR_2)_2$ wherein $R_1$ is a $C_2$—$C_4$ alkylene radical and $R_2$ is an alkyl radical. Preferred examples of such silicates are $Si(O—C_2H_4—N(CH_3)_2)_4$, $Si(OC_2H_4N(C_2H_5)_2)_4$, the two $Si(O—C_3H_6—N(CH_3)_2)_4$ isomers, $Si((O—C_2H_4)_2—NCH_3)_2$ and $Si((O—C_2H_4)_2—NC_2H_5)_2$.

The catalysts themselves may be conveniently prepared from a lower alkyl orthosilicate, e.g. $Si(OCH_3)_4$ or $Si(OCH_2CH_2CH_2CH_3)_4$ and the appropriate alkanolamine by a transesterification reaction. Thus, $Si(O—C_2H_4—N(CH_3)_2)_4$ can be prepared from tetramethyl orthosilicate and N,N-dimethylethanolamine $Si(OCH_3)_4 + 4(CH_3)_2NC_2H_4OH \rightarrow Si(O—C_2H_4N(CH_3)_2)_4 + 4CH_3OH$.

The reaction which forms isocyanurate or isocyanurate linkages can occur at room temperature but in certain cases it may be desirable to work at elevated temperature to increase reaction rates. Conveniently the reaction is carried out in the temperature range from room temperature to 150°C, preferably in the range from room temperature to 100°C.

By the term isocyanate condensation polymers is meant polymers which are derived either exclusively from a polyfunctional isocyanate monomer or from a polyfunctional isocyanate monomer in combination with a second polyfunctional monomer. The second monomer may be, for example a polyol, a polymer polyol, or a polyepoxide. When the second monomer is a polyol or polymer polyol, it is possible to produce a polyurethane containing isocyanurate linkages, whilst if the second monomer is a polyepoxide poly(2-oxazolidones) containing isocyanurate linkages can be produced. The presence of such isocyanurate linkages in the polymer product can confer favourable physical properties, such as mechanical strength and resistance to smoulder, on the product.

Methods for producing such polyols as well known and the skilled man will be familar with the addition of additives such as blowing agents or surfactants to improve the reaction or modify the physical characteristics of the final polymer. In particular techniques which can be used to prepare the polymer in a variety of physical forms, e.g. as an elastomer, resin, foam or fibre, are contemplated as being used in conjunction with the present invention.

The present invention is now illustrated by the following examples.

Example 1

This example illustrates the use of the silicate catalysts to catalyse the trimerisation of isocyanates.

0.1 moles of phenyl isocyanate, 0.01 moles of $Si(OC_2H_4N(CH_3)_2)_4$ and 40 mls of benzene were heated to reflux for 5 hours. At the end of 5 hours, the reaction mixture was cooled and the product allowed to crystallise from solution overnight. Analysis of the product by melting point revealed that it was triphenyl isocyanurate (mp=282—284°C). The yield of product was 63%.

Example 2

The method of example 1 was followed using $Si((OC_2H_4)_2NCH_3)_2$ as the catalyst. Again triphenyl isocyanurate was formed at a yield of 78.2%.

Examples 3—7

These examples illustrate the preparation of flexible polyurethane foams containing isocyanurate linkages from TDI (toluene diisocyanate) and Niax® 11—34 and 34—28 (polyfunctional alcohols).

| COMPONENT (g) | EXAMPLE 3 | 4 | 5 | 6 | 7 |
|---|---|---|---|---|---|
| Niax®11-34 | 30 | 30 | 30 | 30 | 30 |
| Niax®34-28 | 20 | 20 | 30 | 20 | 20 |
| Surfactant SC 152 | 0.5 | 1.0 | 1.0 | 1.0 | 1.0 |
| Water | 1.5 | 1.5 | 1.5 | 1.5 | 1.5 |
| $Si(OC_2H_4N(CH_3)_2)_4$ | 0.5 | 0.5 | 0.5 | 0.5 | 0.5 |
| T-12 | 0 | 0.1 | 0 | 0 | 0 |
| DABCO®33LV | 0 | 0 | 0 | 0.1 | 0 |
| NIAX®A-1 | 0 | 0 | 0 | 0 | 0.1 |
| TDI (80/20) | 22.2 | 22.2 | 22.2 | 22.2 | 22.2 |
| TDI INDEX | 130 | 130 | 130 | 130 | 130 |
| PRODUCT | | | | | |
| Cream time (secs) | 15 | 30 | 14 | 20 | 18 |
| Rise time (secs) | 130 | 129 | 198 | 185 | 95 |
| Tack free time (secs) | 450 | 438 | 500 | 425 | 370 |
| Density | 63.8 | 34.1 | 52.6 | 38.8 | 35.2 |
| Flammability (TG ASTM D 1592-68 | 90 | 59 | 105 | 80 | 77 |

Example 8

A poly(2-oxazolidone) foam with isocyanurate linkages was prepared as follows

26 g of the diglycidyl ether of bisphenol A, 30 g of $CCl_3F$, 2 g of silicone surfactant DC—193, 6 g of $Si(OC_2H_4N(CH_3)_2)_4$ and 100 g of PAPI® 135 (a polymeric isocyanate were stirred vigorously. This mixture had an NCO:epoxy ratio of 5:1. The foam produced had the following properties.

foam density = 39.6 kg/m$^3$

Butter chimney test result = 91.2% wt retention

Tumbling friability = 88%

The mixture which had a cream time of 18 secs and a rise time of 14 mins produced a polymer which, by infrared spectroscopy, was shown to exhibit both isocyanurate and 2-oxazolidone linkages.

**Claims**

1. A process for the preparation of an isocyanurate from an isocyanate or mixture of isocyanates by reacting the isocyanate or mixture thereof in the presence of a silicate catalyst having the formula

$$Si(O-R_1-N(R_2)_2)_4 \quad 1)$$

or

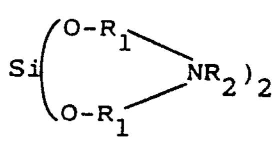

2)

wherein $R_1$ is a $C_2$—$C_4$ alkylene radical and

$R_2$ is an alkyl radical.

2. A process for the preparation of isocyanate condensation polymers containing isocyanurate linkages by reacting a polyfunctional isocyanate with a polyfunctional monomer, under conditions which cause polymerisation, in the presence of a silicate catalyst having the formula

$$Si(O-R_2-N(R_2)_2)_4 \qquad 1)$$

or 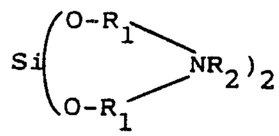 2)

wherein $R_1$ is a $C_2$—$C_4$ alkylene radical and $R_2$ is an alkyl radical.

3. A process as claimed in Claim 2 wherein the isocyanate condensation polymer is a polyurethane and the polyfunctional monomer is either a polyol or polymer polyol.

4. A process as claimed in Claim 2 wherein the isocyanate condensation polymer is a poly(2-oxazolidone) and the polyfunctional monomer is a polyfunctional epoxide.

5. A process as claimed in Claim 1 wherein the isocyanate is a polyfunctional isocyanate and the isocyanate condensation polymer formed is a polyisocyanurate.

6. A process as claimed in Claim 1 or 2 wherein the silicate catalyst is selected from the group comprising $Si(OCH_2CH_2N(CH_3)_2)_4$, $Si(OCH_2CH_2N(C_2H_5)_2)_4$, $Si((OCH_2CH_2)_2NCH_3)_2$ and $Si((OCH_2CH_2)_2NC_2H_5)_2$.

7. Isocyanate condensation polymers containing isocyanurate linkages produced by a process as claimed in Claim 2.

## Patentansprüche

1. Verfahren zur Herstellung eines Isocyanurates aus einem Isocyanat oder einer Mischung von Isocyanaten durch Umsetzung des Isocyanates oder der Mischung davon in Anwesenheit eines Silikatalysators der Formel

$$Si(O-R_1-N(R_2)_2)_4 \qquad 1)$$

oder 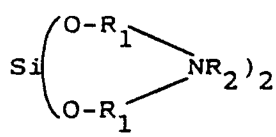 2)

worin $R_1$ eine $C_2$—$C_4$ Alkylenrest und $R_2$ ein Alkylrest sind.

2. Verfahren zur Herstellung von Isocyanat-Kondensationspolymeren, enthaltend isocyanurat-Bindungen durch Umsetzung eines polyfunktionellen Isocyanates mit einem polyfunktionellen Monomer unter Polymerisationsbedingungen in Anwesenheit eines Silikatkatalysators der Formel

$$Si(O-R_1-N(R_2)_2)_4 \qquad 1)$$

oder 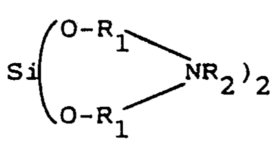 2)

worin $R_1$ eine $C_2$—$C_4$ Alkylenrest und $R_2$ ein Alkylrest sind.

3. Verfahren nach Anspruch 2, worin das Isocyanat-Kondensationspolymer ein Polyurethan und das polyfunktionelle Monomer entweder ein Polyol oder ein Polymerpolyol ist.

4. Verfahren nach Anspruch 2, worin das Isocyanat-Kondensationspolymer ein Poly(2-oxazolidon) und das polyfunktionelle Monomer ein polyfunktionelles Epoxyd ist.

5. Verfahren nach Anspruch 1, worin das Isocyanat ein polyfunktionelles Isocyanat und das gebildete Isocyanat-Kondensationspolymer ein Polyisocyanurat ist.

6. Verfahren nach Anspruch 1 oder 2, worin der Silkatkatalysator ausgewählt ist aus der Gruppe umfassend $Si(OCH_2CH_2N(C_3)_2)_4$, $Si(OCH_2CH_2N(C_2H_5)_2)_4$, $Si((OCH_2CH_2)_2NCH_3)_2$ und $Si((OCH_2CH_2)_2NC_2H_5)_2$.

7. Isocyanat-Kondensationspolymere enthaltend Isocyanurat-Bindungen hergestellt durch ein Verfahen nach Anspruch 2.

## Revendications

1. Procédé de préparation d'un isocyanurate à partir d'un isocyanate, ou à partir d'un mélange d'isocyanates, par réaction de l'isocyanate, ou de son mélange, en présence d'un catalyseur à base de silicate répondant à la formule:

$$Si(O-R_1-N(R_2)_2)_4 \qquad 1)$$

ou 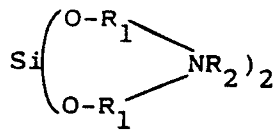 2)

où $R_1$ représente un radical alkylène en $C_2$ à $C_4$ et $R_2$ représente un radical alkyle.

2. Procédé de préparation de polymères de condensation d'isocyanate(s), contenant des liaisons isocyanurates, par réaction d'un isocyanate polyfonctionnel avec un monomère polyfonctionnel, dans des conditions provoquant une polymérisation, en présence d'un catalyseur à base de silicates répondant à la formule:

$$Si(O-R_2-N(R_2)_2)_4 \qquad 1)$$

ou 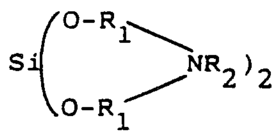 2)

où $R_1$ représente un radical alkylène en $C_2$ à $C_4$, et $R_2$ représente un radical alkyle.

3. Procédé tel que revendiqué à la revendication 2, dans lequel le polymère de condensation d'isocyanate(s) est un polyuréthanne, et le monomère polyfonctionnel est un polyol ou un polyol polymère.

4. Procédé tel que revendiqué à la revendication 2, dans lequel le polymère de condensation d'isocyanate(s) est une poly(2-oxazolidone), et le monomère polyfonctionnel est un époxyde polyfonctionnel.

5. Procédé tel que revendiqué à la revendication 1, dans lequel l'isocyanate est un isocyanate polyfonctionnel, et le polymère de condensation d'isocyanate formé est un polyisocyanurate.

6. Procédé tel que revendiqué à la revendication 1 ou 2, dans lequel le catalyseur à base de silicate est choisi dans l'ensemble constitué par $Si(OCH_2CH_2N(CH_3)_2)_4$, $Si(OCH_2CH_2N(CH_2H_5)_2)_4$, $Si((OCH_2CH_2)_2NCH_3)_2$ et $Si((OCH_2CH_2)_2NC_2H_5)_2$.

7. Polymères de condensation d'isocyanate(s) contenant des liaisons isocyanurates, produits par un procédé tel que revendiqué à la revendication 2.